# EUROPEAN PATENT SPECIFICATION

(11) **EP 2 362 217 B1**
(45) Date of publication and mention of the grant of the patent: **21.09.2016**
(21) Application number: 08879102.5
(22) Date of filing: 22.12.2008
(51) Int. Cl.: G01N 33/20, C21C 5/46, G01F 23/26, F27D 21/00, G01N 27/411, G01F 23/22

(54) **MOLTEN METAL MEASURING SYSTEM WITH PROBE**
MESSSYSTEM FÜR GESCHMOLZENES METALL MIT SONDE
SYSTÈME DE MESURE POUR MÉTAL FONDU AVEC SONDE

(43) Date of publication of application: 31.08.2011
(73) Proprietor: Kawaso Electric Industrial Kabushiki Kaisha, Osaka 550-0005 (JP)
(72) Inventor: AWAZU, Yoshiyuki, Osaka-shi, Osaka 550-0005 (JP); MATSUMOTO, Kouzo, Osaka-shi, Osaka 550-0005 (JP); HATAZAKI, Yutaka, Osaka-shi, Osaka 550-0005 (JP)
(74) Representative: Hering, Hartmut
(86) International application number: PCT/JP2008/073289
(87) International publication number: WO 2010/073304

(56) References cited:
- EP-A1- 1 813 919
- JP-A- 4 348 230
- JP-A- 6 229 902
- JP-A- H04 348 230
- KR-A- 20020 064 527
- US-A- 4 003 260

## Description

### [Field of the Invention]

The present invention relates to a molten metal measuring system which measures elements to be measured of molten metal by immersing a probe in molten metal wherein the probe can be stopped at a predetermined depth position in molten metal from its level.

### [Background of the Invention]

Conventionally, a system, which measures elements to be measured of molten metal with a probe, comprises a sub-lance of which lower end is connected to upper end of the probe, a drive device which makes the sub-lance move up and down, and a control device which controls the drive device for making the sub-lance move down, stop and move up.

The probe, which is provided with measuring means such as a temperature sensor, an oxygen sensor, CD (carbon determination) sensor, a sampler or the like for measuring the elements to be measured of molten metal, is immersed in molten metal by moving the sub-lance downward. The temperature sensor measures the temperature of molten metal. The oxygen sensor is publicly known as disclosed in the Japanese Patent No. 4,017,207 granted to the present applicant. The oxygen sensor comprises, for example, a tube with its bottom end closed which is formed by solid electrolyte having oxygen ion conductivity, and measures the dissolved oxygen and/or the solute elements in molten metal according to an electromotive force generated by the oxyecoia-partial-pressure difference between the tube inside and the molten metal. The CD sensor measures the amount of carbon contained in molten metal by measuring the temperature of molten metal when it is solidified in the probe. The measurement signals outputted from these sensors are sent to a computer in real time.

The sampler extracts a sample of molten metal. After pulling up the probe from molten metal, the solidified sample is taken out from the sampler. The sample is analyzed by emission spectrochemical analysis, the combustion chemical analysis, or the like.

The control device which controls the drive device of sub-lance is constituted by a program provided in said computer, and gives a downward command, a stop command, and a upward command to the drive device. Usually, as for the downward command, it is programmed in such manner that the sub-lance moves downward in a high speed (150m/min for example) at first, then slows down to a medium speed (20m/min for example), and further slows down to a low speed (8m/min for example) when approaching to an immersion position.

Molten metal in a converter during steelmaking process, for example, waves the level surface violently due to oxygen blowing. Accordingly, it is difficult to make a probe immersed. For this reason, it is usual to make a probe immersed where the blowing is stopped. However, a molten slag layer exists above the level of molten metal. Therefore, in order to determine the desired immersion position of the probe (i.e., the distance from the position where the sub-lance starts moving downward to the position where it is to be stopped), it becomes important to check the position of the molten metal level hidden under the molten slag layer. If the sub-lance is moved downward and stopped at an irrelevant position without checking the molten metal level, the immersion position of the probe may be too shallow, or may be too deep on the contrary.

When the immersion position of the probe is shallower, measuring elements cannot be measured correctly but it becomes a cause of incorrect measurement. For example, the temperature is unstable in a shallow part of molten metal from the level thereof so that an electromotive force of the oxygen sensor may become unstable, or an extraction of molten metal sample by the sampler may become poor.

When the immersion position of the probe is deeper on the contrary, the sub-lance approaches too closely to the molten slag layer and may be damaged. For example, there are possibilities that spattering drops of molten slag or molten metal may adhere to the sub-lance, and the sub-lance may be bent due to heat affection, or a holder portion connecting the probe may be baked and damaged. The sampler may be fallen out of the probe when the probe is immersed deeply and burnout.

For this reason, it is performed conventionally that the position of the molten metal level is detected in advance of immersion of the probe, and then the probe is immersed to a predetermined immersion depth position. A device for detecting the molten metal level is known which comprises a pair of electrodes.

Such an electrode-type device is moved downward to molten metal by descending a sub-lance and detects the molten metal level by energization of the electrodes upon contact to the molten metal level. However, since a molten slag layer exists above the molten metal level, the electrodes energize when contacting to such molten slag layer. Therefore, even if the device can detect the position of slag level with energization of the electrodes, but it cannot detect the position of the molten metal level underneath the slag layer. For this reason, it is performed at the present that the position of the slag level is detected at first, and then the position of the molten metal level is determined by calculating the thickness of the slag layer based on the experimental data.

Patent document 1: Japanese patent publication-A No. 2001-50796 The prior art document JP 4-348230 A discloses two coils generating an electromotive force induced by molten metal.

KR20020064527 discloses a method of determining a slag thickness and measuring temperature with one probe.

### [Description of the Invention]

### [Problems Discovered by the Invention]

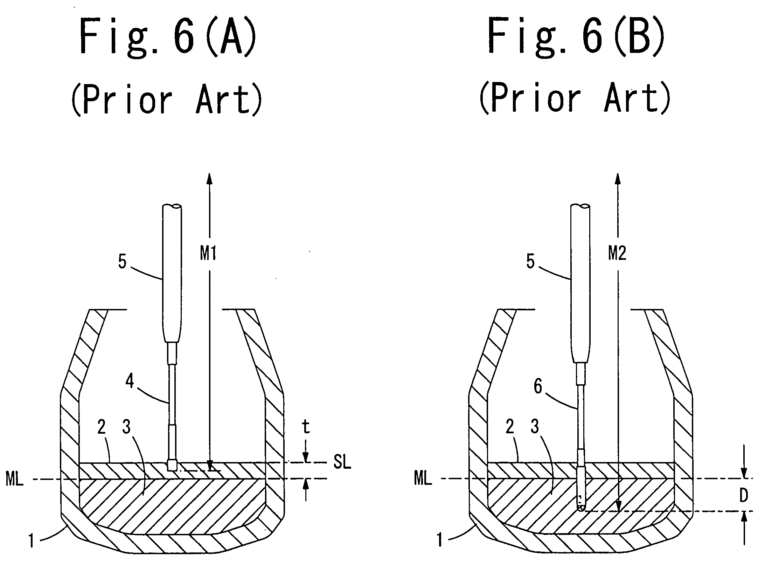

Conventional technology is explained based on drawings. As shown in Fig.6 (A), molten metal 3 has molten slag layer 2 on the surface in a furnace 1. Detection of the level (ML) of molten metal 3 is performed by using a level measuring device 4 under the state that blowing is stopped. The level measuring device 4 is provided with a pair of electrodes as mentioned above, and is moved down by a sub-lance 5. Upon contact to the molten slag layer 2 as illustrated, the electrodes energize so that the position of the molten slag level (SL) is detected. Then, downward migration distance (M1) of the sub-lance 5 is recorded.

Next, a probe 6 is attached to the sub-lance 5 pulled up and is moved downward until the lower end of probe 6 is immersed in a predetermined immersion depth area (D) from the molten metal level (ML) as shown in Fig.6 (B), and the probe 6 is stopped there. The downward migration distance (M2) of the sub-lance 5 is determined based on the slag level (SL) detected previously and the thickness (t) of the slag layer 2 which may be experientially presumed.

In the prior art mentioned above, the position of the level (SL) of molten slag layer 2 is measured by descending, stopping and moving up the sub-lance 5 to which the level measuring device 4 is attached. Then, the probe 6 is attached to the sub-lance 5 while it is pulled up, and the sub-lance 5 is again descended, stopped and moved up for measuring desired elements to be measured by the probe. Therefore, a relatively long time is needed from the start of work to an end. Since blowing must be stopped during such prolonged work, there is a problem of decreasing the productivity of steel manufacture.

Further, the downward migration length (M2) of sub-lance 5, by which the probe 6 was made immersed, includes lower reliability since it is determined according to the experimental result of presuming layer thickness (t) of the slag layer 2. Therefore, even though it is intended to immerse the lower end of the probe 6 in the predetermined immersion depth area (D) from the metal level (ML), it may be actually too deeper or too shallower than the prescribed depth area (D). Moreover, there is a problem that it cannot be checked whether the immersion to the prescribed depth area (D) has been realized.

In addition, it is costly since the level measuring device 4 is consumed at every time.

### [Means for Solving the Problems]

It is an object of the invention to provide a molten metal measuring system with a probe used therein by which the problems mentioned above are solved. According to an aspect of the invention, there is provided a molten metal measuring system in which elements to be measured in molten metal are measured by immersing a probe, which is movable up and down, in molten metal. The system comprises a sub-lance connecting its lower end to a upper end portion of said probe, a drive device for making said sub-lance move up and down, and a control device for controlling said drive device to make said sub-lance move downward, stop moving and move upward. Said probe forms an arranged immersion portion in a predetermined depth area (D) provided in a lower end portion thereof which may be immersed in molten metal by downward movement of the probe at a speed (V). Said arranged immersion portion provides measuring means for measuring elements to be measured of molten metal, and a metal detection sensor in the position at a distance (Dx) below from the upper end (U) of the predetermined depth area (D) which outputs a detection signal (DS) when entering the metal level (ML) of molten metal after passing through the molten slag layer. A data creation device is provided between said metal detection sensor of the probe and said control device. Said data creation device includes stop signal output means which outputs, upon detection of said detection signal (DS), a stop signal (SS) simultaneously with an arrival of a stop time point (T2) which comes after passing a downward continuation time period (Tx) (Tx=Dx/V) from a detection time point (T1) determined by detecting said detection signal (DS) of the metal detection sensor. Said stop signal (SS) is inputted into said control device so that the sub-lance is stopped moving in the position where the upper end (U) of the arranged immersion portion of the probe coincides with the metal level (ML) of molten metal.

According to the invention, judging means is provided between said metal detection sensor and said data creation device. Said judging means receives said detection signal (DS) and compares variation thereof with a predetermined threshold. A detection determination signal (JS) is inputted into said data creation device when said judging means judges the value of said detection signal exceeds said threshold.

Said data creation device includes stop position determination means. Said stop position determination means detects said detection time point (T1) based on the output of said detection signal (DS) from the metal detection sensor, and determines said stop time point (T2) by seeking said downward continuation time period (Tx) (Tx=Dx/V) from said detection time point (T1).

Setting means may be provided in said data creation means. Said setting means may be constituted to set a value of said distance (Dx) and input it into said stop position determination means. Said setting means may also be constituted to set a value of said downward continuation time period (Tx) and input it into said stop position determination means.

It is preferred that said data creation device provides level position data creating means which creates information concerning metal level of molten metal based on said detection signal (DS) of the metal detection sensor.

Preferably, said data creation device provides immersion position data creating means which creates, by seeking a distance from the metal level to the immersion position of said measuring means based on said stop time point (T2), information concerning depth position where said elements to be measured have been measured by measuring means.

Display means may be provided in said data creation device so that information recorded in said level position data creating means or in said immersion position data creating means may be displayed on a display panel.

The metal detection sensor provided in the arranged immersion portion of the probe comprises an LC oscillating circuit. Said LC oscillating circuit usually outputs predetermined voltage, but causes a voltage reduction by reacting to an eddy current which is produced in molten metal when the circuit approaches closely to molten metal.

In the probe, said measuring means provided in the arranged immersion portion includes one or more selected from a temperature sensor for measuring the temperature of molten metal, an oxygen sensor for measuring the dissolved oxygen and/or the solute elements contained in molten metal, a CD sensor measuring the solidifying temperature of molten metal, and a sampler.

According to a preferred embodiment of the invention, the probe provides slag sampling means in a predetermined region (d) which is located above from the upper end (U) of said arranged immersion portion at a distance (dx) which is formed under the condition of dx<t with respect to a thickness (t) of the molten slag layer. Therefore, said slag sampling means is immersed in the molten slag layer in a position separate above from the molten metal level (ML) at said distance (dx), in a state that the probe is immersed in molten metal to make upper end (U) of the arranged immersion portion coincides with the molten metal level (ML).

### [Brief Description of the Drawings]

[Fig.1]

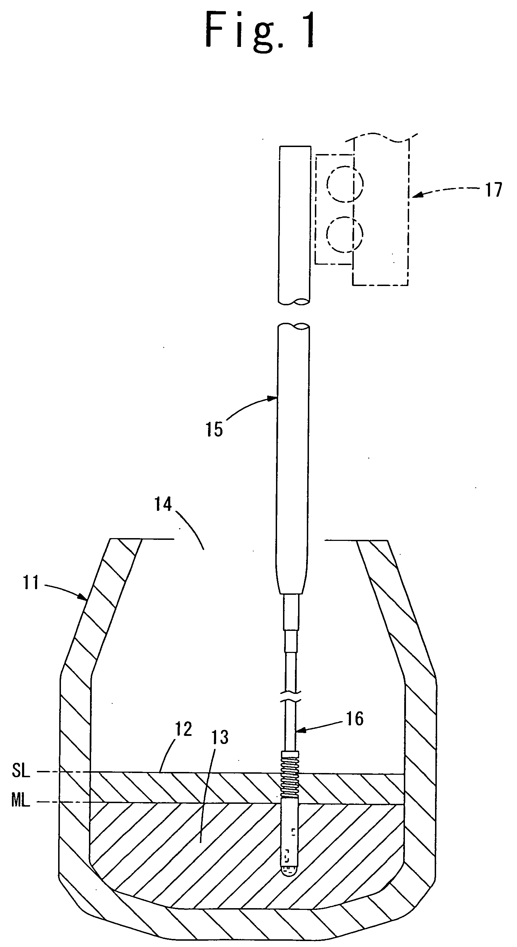

   Fig.1 is a sectional view showing a measuring process of elements to be measured of molten metal according to a measuring system of the invention.
[Fig.2]

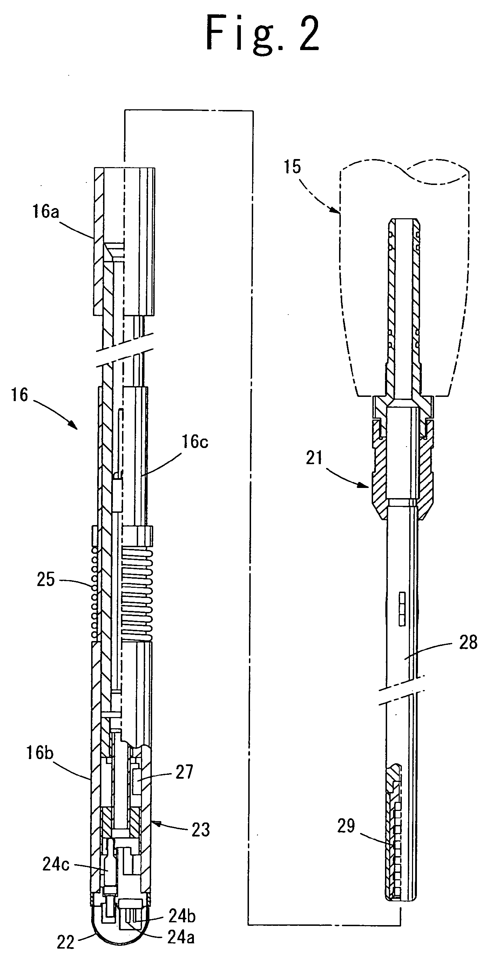

   Fig.2 is a sectional view showing a probe and a holder of a sub-lance used in a measuring system of the invention.
[Fig.3]

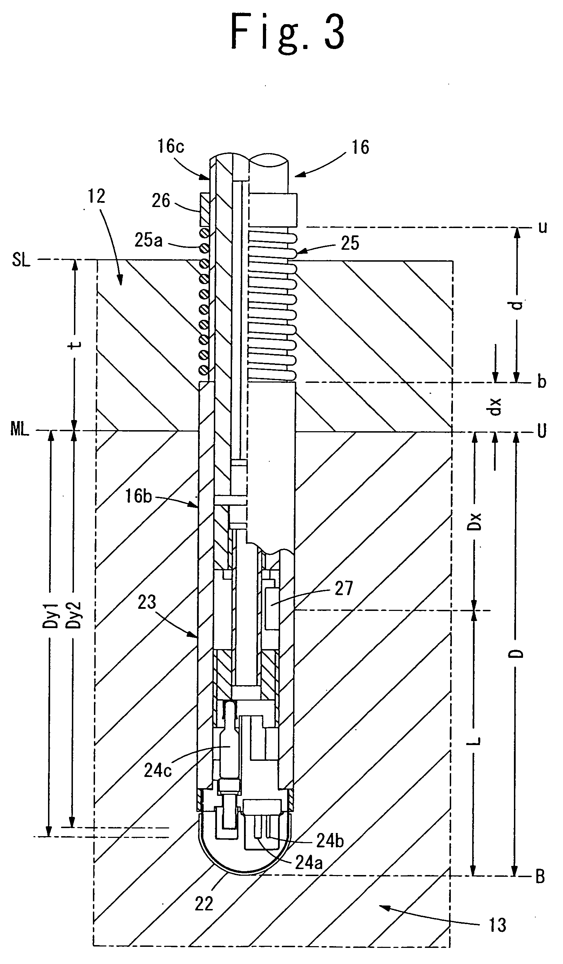

   Fig.3 is a sectional view showing a probe in a state that it is immersed in molten metal according to a measuring system of the invention.
[Fig.4]

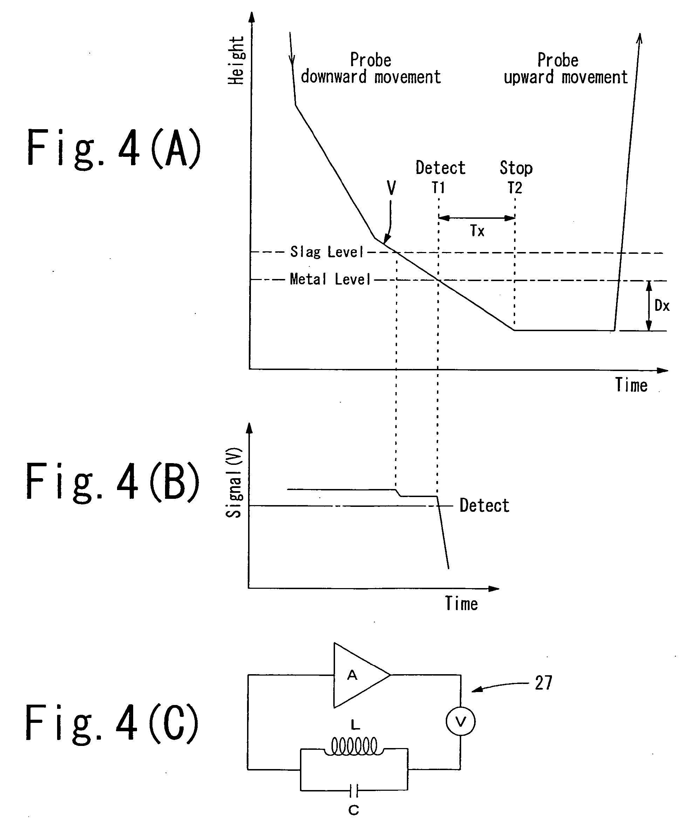

   Fig. 4 shows how to stop a probe during its downward movement according to a measuring system of the invention; (A) is a diagram showing a relationship between downward movement of a probe and its time, (B) is a diagram showing a relationship between a reaction of a metal detection sensor and its time, and (C) is a circuit diagram showing one example of a metal detection sensor.
[Fig.5]

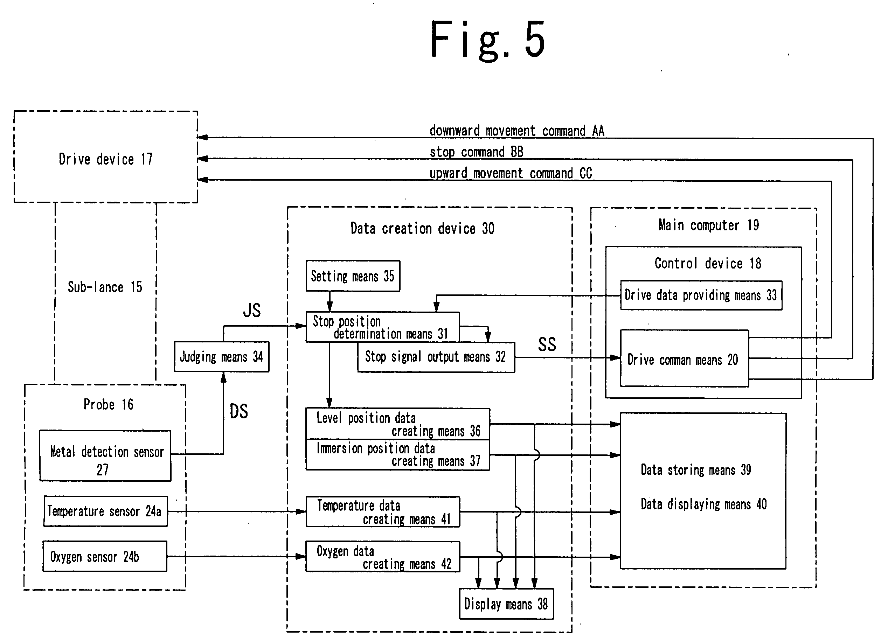

   Fig.5 is a block diagram showing a whole arrangement of a measuring system of the invention.
[Fig.6]
   Fig. 6 shows conventional technology; (A) is a sectional view showing a state where a molten metal level is detected with the level measuring device, and (B) is a sectional view showing a state where elements to be measured of molten metal are measured with a probe.

### [Description of Reference Numerals]

- 11: converter
- 12: molten slag layer
- 13: molten metal
- 15: sub-lance
- 16: probe
- 17: drive device
- 18: control device
- 19: main computer
- 20: drive command means
- 21: holder
- 23: arranged immersion portion
- 24: measuring means
- 24a: temperature sensor
- 24b: oxygen sensor
- 24c: sampler
- 25: slag sampling means
- 27: metal detection sensor
- 30: data creation device
- 31: stop position determination means
- 32: stop signal output means
- 33: drive data providing means
- 34: judging means
- 35: setting means
- 36: level position data creating means
- 37: immersion position data creating means
- 38: display means
- 41: temperature data creating means
- 42: oxygen data creating means

### [Preferred Embodiments of the Invention]

A detailed description will be given of preferred embodiments of the invention in reference to drawings as follows.

Fig.1 illustrates a system which measures elements to be measured of molten metal which is under refinement with converter 11. The molten metal 13 has molten slag layer 12 on the surface, and is refined by blowing of oxygen. The elements to be measured of molten metal are measured in such manner that a probe 16 attached to a sub-lance 15 is moved downward through upper opening 14 of the converter 11 and is immersed in a predetermined depth position of molten metal 13, where the blowing is in abeyance.

The measuring system comprises said probe 16, said sub-lance 15 of which lower end portion is connected to upper end portion of the probe 16, a drive device 17 which makes the sub-lance 15 move up and down, and a control device 18 which controls the drive device 17 for making the sub-lance 15 move down, stop moving and move up. As shown in Fig.5, the control device 18 is constituted by a program provided in a main computer 19, and is provided with drive command means 20 which gives a downward command, a stop command, and a upward command to the drive device. Usually, as for the downward moving speed of the sub-lance 15, it is programmed in such manner that the sub-lance 15 moves downward in a high speed (150m/min for example) at first, then slows down to a medium speed (20m/min for example), further slows down to a low speed (8m/min for example) when approaching to the slag layer 12, then stops moving at the predetermined descending position for 15 (fifteen) seconds, and moves upward in a high speed.

The elements to be measured of molten metal which the probe 16 aims to measure 16 are the temperature of molten metal, the dissolved oxygen and/or the solute elements contained in molten metal, and the carbon in molten metal. Further, the probe 16 aims to extract a sample of molten metal.

In addition to the purposes of measuring such elements of molten metal, the illustrated probe 16 aims to extract the slag. An extracted slag sample offers important information for a steel-manufacture process by analyzing the sample with a combustion chemical analysis or the like. For this reason, extracting of slag sample has the strong needs from steel-manufacture factories. However, such slag sample needs to extract sufficient quantity which can be analyzed. If molten metal is mixed in the extracted slag sample, analysis will become difficult or impossible. Accordingly, it is important, when extracting slag, that sufficient quantity of a sample is extracted and no molten metal is mixed in the extracted sample.

As shown in Fig.2, the probe 16 is formed by paper tubes in which a upper end portion 16a and a lower end portion 16b of thick tubes respectively are connected by an extension tube 16c which is thinner than said tubes. The upper end portion 16a of the probe 16 may be attached detachably to a holder 21 provided at lower end of the sub-lance 15.

As shown in Fig.3, the lower end portion 16b of the probe 16 provides an arranged immersion portion 23 corresponding to the predetermined immersion depth area (D) between the lower end (B) including a metal protection cap 22 and the upper end (U) thereof. Measuring means 24 for measuring the elements to be measured of molten metal 13 is equipped in the arranged immersion portion 23. The measuring means 24 includes one or more means selected from a temperature sensor 24a for measuring the temperature of molten metal, an oxygen sensor 24b for measuring the dissolved oxygen and/or the solute elements contained in molten metal, a CD (carbon determination) sensor (not shown) for measuring the solidifying temperature of molten metal, a sampler 24c, and other means. In the illustrated example, the temperature sensor 24a and the oxygen sensor 24b are located at a distance (Dy1) below from the upper end (U) of the predetermined immersion depth area (D). The sampler 24c arranges its opening for extracting molten metal at a distance (Dy2) above from the upper end (U) of the predetermined immersion depth area (D).

The probe 16 forms a predetermined region (d) which is separated a predetermined distance (dx) above from the upper end (U) of the arranged immersion portion 23, and provides slag sampling means 25 thereon. In the illustrated example, the slag sampling means 25 comprises a metal coil 25a wound around the predetermined region (d) of the extension tube 16c which is located right above the lower end portion 16b, and the coil 25a forms a spiral gap between neighboring circles thereof. A fixing ring 26 is secured on the extension tube 16c and is engaged with upper end of the coil 25a.

By immersing the arranged immersion portion 23 in a predetermined depth of molten metal 13, the temperature sensor 24a measures the temperature of molten metal. The oxygen sensor 24b may be as same as a sensor proposed by the present applicant in the Japanese patent No. 4017207 that comprises a tube with its bottom end closed which is formed by solid electrolyte having oxygen ion conductivity, and an external electrode. The dissolved oxygen and/or the solute elements contained in molten metal may be measured according to an electromotive force generated by the oxyecoia-partial-pressure difference between the tube inside and the molten metal. The CD sensor, which is not illustrated, measures the amount of carbon contained in molten metal by measuring the solidifying temperature of molten metal which is extracted by a vessel provided in the probe.

The sampler 24c extracts a sample of molten metal 13. A solidified sample is taken out of the sampler 24c after pulling up the probe 16 from molten metal 13. The sample is offered for emission spectrochemical analysis, combustion chemical analysis or the like.

The slag sampling means 25 extracts a sample of molten slag 12 during immersion thereof in the molten slag layer 12 in such manner that molten slag is made to enter in the spiral gap of the coil 25a and adhere around periphery of the coil 25a. The extracted slag is offered as a solidified slag sample for combustion chemical analysis or the like after pulling up the probe 16. The slag sample needs not to be mixed with molten metal as mentioned above.

According to the invention, a metal detection sensor 27 is provided in the arranged immersion portion 23 of the probe 16 which is located at a distance (L) from the lower end (B). As shown in Fig.4 (C), the metal detection sensor 27 is constituted by a circuit such as LC oscillating circuit which outputs predetermined voltage in a stable state. LC parallel oscillating circuit is used in the illustrated example.

The holder 21 of the sub-lance 15 provides a connecting rod 28, as shown in Fig.2. The connecting rod 28 provides a connector 29 which is electrically connected, when inserted in the probe 16, to those sensors included in the measuring means 24 (the temperature sensor 24a, the oxygen sensor 24b, and the CD sensor), and also electrically connected to the metal detection sensor 27. The connector 29 is connected to wiring which leads inside the sub-lance 15, and is further connected to a data creation device 30 illustrated in Fig.5 by another wiring arranged outside. The data creation device 30 is connected to a main computer 19 via interface.

The object of the metal detection sensor 27 according to the invention is to make the sub-lance 15 stop and stay in a position where the upper end (U) of the arranged immersion portion 23 coincides with the molten metal level (ML) as shown in Fig.3, during a process of moving the sub-lance 15 downward by the drive device 17 so that the arranged immersion portion 23 of the probe 16, which is attached to the sub-lance, is immersed in molten metal 13 after passing through the slag layer 12.

At the position where the upper end (U) of the arranged immersion portion 23 is coincided with the molten metal level (ML), the slag sampling means 25 is made to immerse in the molten slag layer 12 in a position separate above from the molten metal level (L) at said distance (dx). The distance (dx) is made enough far from the molten metal level (ML) and is formed under the condition of dx<t with respect to the thickness (t) of the molten slag layer 12. In the illustrated example, the predetermined region (d), in which the slag sampling means 25 is provided, is formed so that it may become d≧t-dx. Accordingly, when the probe 16 is stopped moving so that the upper end (U) of the arranged immersion portion 23 may be coincided with the molten metal level (ML) as mentioned above, the slag sampling means 25 makes its upper end (u) stay at a position upper than the molten slag level (SL) and makes its lower end (b) immerse in the molten slag layer 12 in a position separated said distance (dx) from the molten metal level (ML). In this aspect, if the distance (dx) is formed, for example, under the condition of dx<t/2 or dx<t/3, the slag sampling means 25 can be made immersed over 1/2 or 2/3 of the thickness (t) of the molten slag layer 12.

When the probe 16 is moved downward with the sub-lance 16, the probe 16 enters in molten metal 13 after passing through the slag layer 12. The LC parallel oscillating circuit constituting the metal detection sensor 27, which outputs predetermined voltage, makes the molten metal 13 generate an eddy current at the same time when it contacts the molten metal level (ML). In response to such eddy current, the output voltage of metal detection sensor 27 declines rapidly. This voltage reduction is used as a detection signal of detecting the metal level (ML).

In the illustrated example, the predetermined immersion depth area (D) (the length between the lower end (B) and the upper end (U)) and the position (L) where the metal detection sensor 27 is provided (distance from the lower end (B)) are formed under the condition of D≧L. The distance between the metal detection sensor 27 and the upper end (U) is formed under the condition of D-L=Dx. Accordingly, the upper end (U) of the arranged immersion portion 23 becomes coincide with the molten metal level (ML) and stays there, when the probe 16 is made to stop after moving downward to the depth (Dx) from the position where the metal detection sensor 27 has detected the metal level (ML).

As showing the time flow in Fig.4 (A), a detection time point (T1) is determined when the metal detection sensor 27 outputs a detection signal by detecting the metal level (ML) of molten metal 13 while the probe 16 moves downward at speed V A stop time point (T2) is determined by seeking downward continuation time period Tx (Tx=Dx/V) from the detection time point (T1). If the downward movement is stopped at the stop time point (T2), the probe 16 will stop in the position where the metal detection sensor 27 is immersed in the depth (Dx) from the metal level (ML). As a result, the probe 16 stops and stays in the position where the upper end (U) of the arranged immersion portion 23 coincides with the metal level (ML).

In order to perform the operation as mentioned above, as shown in Fig.5, the data creation device 30 is provided with stop position determination means 31 for determining the downward continuation time period (Tx), and is provided with stop signal output means 32 for outputting a stop signal to the drive command means 20 of the control device 18. Drive data providing means 33 is included in the control device 18 to input information such as the speed (V) etc. into the stop position determination means 31.

Preferably, judging means 34 is provided between the metal detection sensor 27 and the stop position determination means 31. The judging means 34 outputs a detection determination signal (JS) to the stop position determination means 31, when the voltage reduction of the detection signal (DS) inputted from the metal detection sensor 27 is judged as a trigger signal exceeding a threshold to be compared. Therefore, the judging means 34 does not output the detection determination signal (JS), unless the voltage reduction exceeds the threshold as shown in Fig.4 (B), even when the output voltage of metal detection sensor 27 declines upon receipt of any electric influence when passing through the molten slag layer 12. On the other hand, the judging means 34 outputs the detection determination signal (JS) when the metal detection sensor 27 reaches the molten metal level (ML), since the output voltage falls rapidly and exceeds the threshold greatly.

When the metal detection sensor 27 enters the molten metal level (ML) and outputs the detection signal (DS), the stop position determination means 31, upon receipt of the detection determination signal (JS) from the judging means 34, determines the stop time point (T2) by seeking the downward continuation time period (Tx) (Tx=Dx/V) from the detection time point (T1), and makes the stop signal output means 32 output a stop signal (SS) to the drive command means 20 at the time when the stop time point (T2) is up. The drive command means 20 outputs a stop command and makes the drive device 17 stop. In case that the metal detection sensor 27 is provided at the upper end (U) unlike the illustrated example, the depth may be set up in Dx=0, therefore Tx=0, and the stop signal is outputted simultaneously with outputting of the detection determination signal (JS).

As mentioned above, the probe 16 is made to move downward with the sub-lance 16, to immerse in the molten metal 13 after passing through the molten slag layer 12, and to stop at a position where the upper end (U) of the arranged immersion portion 23 coincides with the metal level (ML). Then, the measuring means 24 measures elements to be measured of the molten metal 13. The temperature sensor 24a measures the temperature of molten metal under the state that it is positioned correctly in the desired depth. The oxygen sensor 24b measures the dissolved oxygen and/or the solute elements contained in molten metal. The CD sensor measures the coagulation temperature of molten metal. The sampler 24c extracts the sample of molten metal.

The probe 16 stops in the position where the upper end (U) of the arranged immersion portion 23 coincides with the metal level (ML) without being immersed too deep in molten metal 13. As a result, the lower end (lower end of connecting rod 28, for example) of sub-lance 15 is enough separated upward from the level (SL) of the molten slag layer 12 so that damages of the sub-lance 15 due to heat affection may be prevented.

If the probe 16 is immersed too deep, molten metal may mix in the slag sample which is extracted by the slag sampling means 25. In comparison, according to the invention, the slag extraction is carried out suitably, since the lower end (b) of the slag sampling means 25 is immersed in the molten slag layer 12 in the position separate above from the metal level (ML) of the molten metal 13 at said suitable distance (dx).

If it is necessary to be compatible with various probes 16 in which dimensions (Dx) or full lengths of probes are different, setting means 35 may be provided as shown in Fig.5. For example, the setting means 35 can be constituted so that it may set up a value of (Dx), or a value of the downward continuation time period (Tx), and may input these set-up values into the stop position determination means 31.

It is preferred that the data creation device 30 provides level position data creating means 36 and immersion position data creating means 37. Information concerning a stop position of the probe 16, which the stop position determination means 31 has determined, may be utilized advantageously.

The level position data creating means 36 acquires the detection determination signal (JS), which is outputted based on the detection signal of the metal detection sensor 27, from the stop position determination means 31. Thereby, the data concerning a position of the metal level (ML) at the time when actual measurement has been carried out, which includes information of height of the metal level (ML) from the furnace bottom, and information of distance to the metal level (ML) from the position where the sub-lance 15 has started moving, or the like, is created.

The immersion position data creating means 37 acquires information concerning the stop time point (T2) from the stop position determination means 31. Thereby, the data concerning immersion position of the probe 16 in molten metal 13 when actual measurement has been carried out, which includes information of the depth distance (Dy1) from the metal level (ML) where the temperature sensor 24a, the oxygen sensor 24b and the CD sensor have carried out respective measurements, or information of the distance (Dy2) from the metal level (ML) where the sampler 26 has extracted a sample, or the like, is created.

The information created by the level position data creating means 36 and the immersion position data creating means 37 may be displayed on a display panel by display means 38 provided in the data creation device 30, and it is preferred to enable it to monitor in real time from the time when the probe 16 has been immersed in molten metal 13. Such data is stored in data storing means 39 of the main computer 19. It is preferred to display the data on a display panel by data displaying means 40 in the main computer 19, and enable it to print with a printer.

Further, it is preferred for the data creation device 30 to provide temperature data creating means 41, and oxygen data creating means 42.

The temperature data creating means 40 receives output signals of the temperature sensor 24a of the probe 16, and creates, based on such signals, the data concerning the temperature of molten metal. The oxygen data creating means 42 receives output signals of the oxygen sensor 25 of the probe 16, and creates, based on such signals, the data concerning the dissolved oxygen and/or the solute elements contained in molten metal.

The information created by the temperature data creating means 41 and the oxygen data creating means 42 may also be displayed on a display panel by the display means 38 provided in the data creation device 30, and it is preferred to enable it to monitor in real time from the time when the probe 16 has been immerses in molten metal 13. Such data is also stored in the data storing means 39 of the main computer 19. It is preferred to display on a display panel by the data displaying means 40 in the main computer 19, and enable it to print with a printer.

### [Industrial Availability]

In order to measure the elements to be measured in molten metal, the upper end portion 16a of the probe 16 is attached to the holder 21 at the lower end of the sub-lance 15, and a downward command is given to the drive device 17 from the control device 18. Downward movement of the sub-lance 15 is carried out by the drive device 17 so that the arranged immersion portion 23 corresponding to the predetermined immersion depth area (D) at the lower end portion 16b of the probe 16 is immersed in molten metal 13. Thereby, the elements to be measured of molten metal 13 are measured by the measuring means 24 provided in the arranged immersion portion 23. For example, the temperature of molten metal 13, the dissolved oxygen and/or the solute elements contained in molten metal 13, and the coagulation temperature of molten metal 13 are measured by the temperature sensor 24a, the oxygen sensor 24b, the CD sensor, the sampler 24c, and others. A sample of molten metal 13 is extracted by the sampler 24c.

The slag sampling means 25 is immersed in the molten slag layer 12, and a slag sample is extracted. The slag sampling means 25 is provided in the predetermined region (d) and is separated above from the upper end (U) of the arranged immersion portion 23 at said distance (dx).

According to the invention, the metal detection sensor 27 is provided in the prescribed position of the arranged immersion portion 23, and the data creation device 30 is provided between the metal detection sensor 27 and the control device 18. The data creation device 30 is provided with the stop position determination means 31 and the stop signal output means 32. Therefore, when the arranged immersion portion 23 of the probe 16 is made to immerse in the molten metal 13, the metal detection sensor 27 outputs the detection signal (DS) at the same time when it advances into the metal level (L) of molten metal 13 after passing through the molten slag layer 12. The stop position determination means 31 determines the stop time point (T2) by seeking the downward continuation time period (Tx) from the detection time point (T1) which is determined based on output of the detection signal (DS). The stop signal output means 32 outputs the stop signal into the control device 18 at the same time when the stop time point (T2) is up, and thereby the drive device 17 is made to stop. The downward continuation time period (Tx) is determined by calculating Tx=Dx/V based on the distance (Dx) between the position of metal detection sensor 27 and the upper end (U) of the predetermined immersion depth area (D) which constitutes the arranged immersion portion 23, and the downward movement speed (V) at the time when the probe 16 is immersed in molten metal 13. Therefore, the sub-lance 15 is made to stop in the position where the upper end (U) of the arranged immersion portion 23 of the probe 16 coincides with the metal level (ML) of molten metal 13.

As a result, the probe 16 always stops at the correct position where the upper end (U) of the arranged immersion portion 23 coincides with the metal level (L) of molten meal 13, without stopping at such positions as too deep or too shallow. Hence, the measuring means 24 can measure the elements to be measured of molten metal in the desired depth from the metal level (ML).

During the probe 16 stops and stays, the slag sampling means 25 is immersed in the molten slag layer 12 in the position above from the metal level (ML) at said distance (dx). Therefore, there is no possibility that molten metal may mix in the slag sample extracted by the slag sampling means 25 so that a desired slag sample may be extracted suitably.

On the other hand, the sub-lance 15 is made to stop in the desired position separate above from the slag level (SL) of the molten slag layer 12 so that the sub-lance 15 may be protected from undesired thermal affection.

The prior art mentioned above requires a step of detecting a molten metal level in advance of carrying out a step of measurement by a probe. In comparison to this, according to the invention, the metal level (ML) of molten metal is detected in the same process of measurement by a probe, and the probe is made to stop in a desired correct immersion depth. Therefore, blowing stop time becomes short, and reduction of the productivity of steel manufacture may be prevented. That is, although the prior art requires tow steps of detection of metal level and subsequent measurement by a probe, it is carried out in one process according to the invention. Also according to the invention, it is advantageous in respect of cost than the prior art which consumes a metal level detecting device in addition to a probe.

The setting means 35 may be provided in the data creation device 30. The setting means 35 sets up the value of the distance (Dx), and the value of the downward continuation time period (Tx) so that these set-up values are inputted into the stop position determination means 31. Thereby, the system of the invention can be used for various probes in which full lengths or dimensions of (Dx) are different.

The metal detection sensor 27 is provided with LC oscillating circuit which outputs predetermined voltage. The LC oscillating circuit reduces the voltage by reacting to an eddy current produced in molten metal, when the circuit approaches to the molten metal 13. The judging means 34 is provided between the metal detection sensor 27 and the stop position determination means 31. The judging means 34 inputs a detection determination signal (JS) into the stop position determination means 31, only when a detection signal (DS) inputted from the metal detection sensor 27, which shows reduction of voltage, is judged as the signal exceeding a predetermined threshold by comparing therewith. Therefore, the judging means 34 does not output the detection determination signal (JS), unless the voltage reduction exceeds the threshold, even if the output voltage of metal detection sensor 27 declines upon receipt of any electric influence when passing through the molten slag layer 12. As a result, false detection of the metal level (ML) may not be carried out inside the molten slag layer 12. Since the metal detection sensor 27 falls the output voltage rapidly exceeding greatly the threshold when it reaches the molten metal level (ML), the judging means 34 can suitably output the detection determination signal (JS) so that the metal level (ML) may be detected correctly.

According to the invention, the data creation device 30 may provide the level position data creating means 36 and the immersion position data creating means. Information, which the stop position determination means 31 has determined, may be utilized advantageously. The immersion position data creating means 36 obtains the output result of the detection determination signal (JS) from the stop position determination means 31 so that information concerning the metal level (L) when actual measurement has been carried out, such as information of height of the metal level (ML) from the furnace bottom, and information of distance to the metal level (ML) from the position where the sub-lance 15 has started moving, or the like, may be created. The immersion position data creating means 37 obtains information concerning the stop time point (T2) from the stop position determination means 31 so that information concerning immersion position of the probe 16 in molten metal 13 when actual measurement has been carried out, such as information of the depth distance from the metal level (ML) where the temperature sensor 24a, the oxygen sensor 24b and the CD sensor have carried out their measurement, or information of the distance from the metal level (ML) where the sampler 26 has extracted a sample, or the like, may be created.

The information created by the level position data creating means 36 and the immersion position data creating means 37 may be displayed on a display panel by display means 38 provided in the data creation device 30, and it may be constituted to enable it to monitor in real time after the probe 16 has been immersed in molten metal 13.

The data creation device 30 may provide the temperature data creating means 41 and the oxygen data creating means 42. The temperature data creating means 40 receives output signals of the temperature sensor 24a of the probe 16, and creates, based on such signals, the data concerning the temperature of molten metal. The oxygen data creating means 42 receives output signals of the oxygen sensor 25 of the probe 16, and creates, based on such signals, the data concerning the dissolved oxygen and/or the solute elements contained in molten metal.

The information created by the temperature data creating means 41 and the oxygen data creating means 42 may also be displayed on a display panel by the display means 38 provided in the data creation device 30, and it may be constituted to enable it to monitor in real time after the probe 16 has been immerses in molten metal 13.

## Claims

1. A molten metal measuring system wherein elements to be measured of molten meal are measured in a predetermined depth area (D) from a metal level (ML) of molten metal (13) on which a slag layer (12) exists, and wherein said system comprises a probe (16) which includes a lower end portion (16b) having an immersed portion (23) to be immersed in said predetermined depth area (D), an upper end portion (16a) to be extended above said slag layer (12) when the probe is immersed, measuring means (24) provided in said arranged immersion portion (23) for measuring elements to be measured of molten meal, and a metal detection sensor (27) located at a position below an upper end (U) of the predetermined depth area (D) at a distance (Dx),
a sub-lance (15) which is connected to the upper end portion (16a) of said probe (16) and with which said probe ( 6) moves downward at a
speed (V) and moves up after stopping the downward movement,
a drive device (17) for making said sub-lance (15) move up and down, and
a control device (18) having drive command means (20) for making said sub-lance (15) move downward, stop moving and move upward by controlling said drive device (18),
**characterized in that**:
said system further comprises a data creation device (30) including stop position determination means (31) which determines, during the downward movement of the probe (16), a detection time point (T1) and a stop time point (T2) which comes after passing a downward continuation time period (Tx) (Tx=Dx/V) from said detection time point (T1), and stop signal output means (32) which outputs a stop signal (SS) to said drive command means (20) simultaneously with an arrival of a stop time point (T2), and
judging means (34) which receives a detection signal (DS) from said metal detection sensor (27) and outputs a detection determination signal (JS) to said stop position determination means (31) for determining said detection time point (T1);
wherein said metal detection sensor (27) includes a circuit which outputs predetermined voltage to said judging means (34) in which the voltage is reduced by reacting to metal in such a manner that the voltage is reduced by reacting to molten slag layer (12) and is further reduced by reacting to molten metal (13) of which voltage reduction constitutes said detection signal (DS), and
judging means (34) receives said detection signal (DS) from said metal detection sensor (27) and compares the voltage reduction thereof with a predetermined threshold, and said judging means (34) outputs a detection determination signal (JS) to said stop position determination means (31) only when the voltage reduction of said detection signal (DS) is judged as exceeding said threshold whereby said stop position determination means (31) determines said detection time point (T1),
thereby said stop position determination means (31) is constituted to determine said detection time point (T1) only when said metal detection sensor (27) reaches to said metal level (ML) of molten meal (13) after passing through said slag layer (12).

2. A molten metal measuring system according to claim 1, wherein setting means (35) is provided in said data creation device (30) so that said setting means (35) sets up values of said distance (Dx) and said downward continuation time period (Tx), and inputs them into said stop position determination means (31).

3. A molten metal measuring system according to claim 1 or 2, wherein said data creation device (30) provides level position data creating means (36) which creates information concerning metal level of molten metal based on said detection signal (DS) of the metal detection sensor (27).

4. A molten metal measuring system according to 1, 2 or 3, wherein said data creation device (30) provides immersion position data creating means (37) which creates, by seeking a distance from the metal level to an immersion position of said measuring means based on said stop time point (T2), information concerning depth position where said elements to be measured have been measured by measuring means.

5. A molten metal measuring system according to claim 3 or 4, wherein display means (38) is provided in said data creation device (30) in which information created by said level position data creating means (36) or by said immersion position data creating means (37) may be displayed on a display panel.

6. A molten metal measuring system according to claim 1, wherein said metal detection sensor (27) provided in the arranged immersion portion (23) of said probe includes a LC oscillating circuit which outputs predetermined voltage but reduces the voltage by reacting to an eddy current which is produced in molten metal when the circuit approaches thereto.

7. A molten measuring system according to claim 1, wherein said measuring means (24) provided in the arranged immersion portion (23) of said probe includes one or more selected from a temperature sensor (24a) for measuring the temperature of molten metal, an oxygen sensor (24b) for measuring the dissolved oxygen and/or the solute elements contained in molten metal, a CD sensor measuring the solidifying temperature of molten metal, and a sampler (24c).

8. A molten measuring system according to claim 1, wherein slag sampling means (25) is provided in a predetermined region (d) of the probe which is located above from the upper end (U) of said arranged immersion portion (23) at a distance (dx) which is formed under the condition of dx<t with respect to a thickness (t) of the molten slag layer (13), thereby said slag sampling means (25) is immersed in the molten slag layer (13) in a position separate above from the molten metal level (ML) at said distance (dx), in a state that the probe is immersed in molten metal to make upper end (U) of the arranged immersion portion (23) coincides with the molten metal level (ML).

## Patentansprüche

1. Messsystem für Metallschmelzen, wobei zu messende Elemente aus geschmolzenem Metall in einem vorbestimmten Tiefenbereich (D) von einer Metallhöhe (ML) von geschmolzenem Metall (13) aus gemessen werden, auf welchem eine Schlackenschicht (12) existiert,
und wobei das System einen Tester (16) aufweist, der einen unteren Endteilbereich (16b) mit einem in den vorbestimmten Tiefenbereich (D) einzutauchenden Eintauchteilbereich (23) enthält, einen oberen Endteilbereich (16a), der sich oberhalb der Schlackenschicht (12) erstrecken soll, wenn der Tester eingetaucht ist, eine in dem arrangierten Eintauchteilbereich (23) vorgesehene Messeinrichtung (24) zum Messen von zu messenden Elementen aus geschmolzenem Metall und einen Metalldetektionssensor (27), der an einer Position unterhalb eines oberen Endes (U) des vorbestimmten Tiefenbereichs (D) mit einem Abstand (Dx) lokalisiert ist,
eine Unterlanze (15), die mit dem oberen Endteilbereich (16a) des Testers (16) verbunden ist und mit welcher sich der Tester (16) mit einer Geschwindigkeit (V) abwärts bewegt und sich nach einem Stoppen der Abwärtsbewegung aufwärts bewegt,
eine Antriebsvorrichtung (17) zum Veranlassen, dass sich die Unterlanze (15) aufwärts und abwärts bewegt, und
eine Steuervorrichtung (18) mit einer Antriebsbefehlseinrichtung (20) zum Veranlassen, dass die Unterlanze (15) sich abwärts bewegt, ein Bewegen stoppt und sich aufwärts bewegt, durch Steuern der Antriebsvorrichtung (18),
**dadurch gekennzeichnet, dass**:
das System weiterhin eine Datenerzeugungsvorrichtung (30) aufweist, die eine Stopppositions-Bestimmungseinrichtung (31) enthält, die während der Abwärtsbewegung des Testers (16) einen Detektionszeitpunkt (T1) und einen Stoppzeitpunkt (T2), der nach einem Verstreichen einer Abwärtsfortdauerzeitperiode (Tx) (Tx=Dx/V) ab dem Detektionszeitpunkt (T1) kommt, bestimmt, und eine Stoppsignal-Ausgabeeinrichtung (32), die gleichzeitig mit einem Eintreffen eines Stoppzeitpunkts (T2) ein Stoppsignal (SS) zu der Antriebsbefehlseinrichtung (20) ausgibt, und
eine Beurteilungseinrichtung (34), die ein Detektionssignal (DS) von dem Metalldetektorsensor (27) empfängt und ein Detektionsbestimmungssignal (JS) zu der Stopppositions-Bestimmungseinrichtung (31) zum Bestimmen des Detektionszeitpunkts (T1) ausgibt;
wobei der Metalldetektionssensor (27) eine Schaltung enthält, die eine vorbestimmte Spannung zu der Beurteilungseinrichtung (34) ausgibt, in welcher die Spannung durch Reagieren mit Metall auf solche Weise reduziert wird, dass die Spannung durch Reagieren mit geschmolzener Schlackenschicht (12) reduziert wird und durch Reagieren mit geschmolzenem Metall (13) weiter reduziert wird, aus welcher Spannungsreduktion sich das Detektionssignal (DS) bildet, und
die Beurteilungseinrichtung (34) das Detektionssignal (DS) von dem Metalldetektionssensor (27) empfängt und die Spannungsreduktion davon mit einer vorbestimmten Schwelle vergleicht und die Beurteilungseinrichtung (34) ein Detektionsbestimmungssignal (JS) zu der Stopppositions-Bestimmungseinrichtung (31) nur dann ausgibt, wenn die Spannungsreduktion des Detektionssignals (DS) derart beurteilt wird, dass sie die Schwelle übersteigt, wodurch die Stopppositions-Bestimmungseinrichtung (31) den Detektionszeitpunkt (T1) bestimmt,
wodurch die Stopppositions-Bestimmungseinrichtung (31) gebildet wird, um den Detektionszeitpunkt (T1) nur dann zu bestimmen, wenn der Metalldetektionssensor (27) nach einem Durchlaufen der Schlackenschicht (12) die Metallhöhe (ML) von geschmolzenem Metall (13) erreicht.

2. Messsystem für Metallschmelzen nach Anspruch 1, wobei eine Einstelleinrichtung (35) in der Datenerzeugungsvorrichtung (30) vorgesehen ist, so dass die Einstelleinrichtung (35) Werte des Abstands (Dx) und der Abwärtsfortdauerzeitperiode (Tx) einstellt und sie in die Stopppositions-Bestimmungseinrichtung (31) eingibt.

3. Messsystem für Metallschmelzen nach Anspruch 1 oder 2, wobei die Datenerzeugungsvorrichtung (30) eine Höhenpositionsdaten-Erzeugungseinrichtung (36) zur Verfügung stellt, die Information in Bezug auf eine Metallhöhe von geschmolzenem Metall basierend auf dem Detektionssignal (DS) des Metalldetektionssensors (27) erzeugt.

4. Messsystem für Metallschmelzen nach Anspruch 1, 2 oder 3, wobei die Datenerzeugungsvorrichtung (30) eine Eintauchpositionsdaten-Erzeugungseinrichtung (37) zur Verfügung stellt, die durch Suchen eines Abstands von der Metallhöhe zu einer Eintauchposition der Messeinrichtung basierend auf dem Stoppzeitpunkt (T2) Information in Bezug auf eine Tiefenposition, wo die zu messenden Elemente durch die Messeinrichtung gemessen worden sind, erzeugt.

5. Messsystem für Metallschmelzen nach Anspruch 3 oder 4, wobei in der Datenerzeugungsvorrichtung (30) eine Anzeigeeinrichtung (38) vorgesehen ist, in welcher durch die Höhenpositionsdaten-Erzeugungseinrichtung (36) oder durch die Eintauchpositionsdaten-Erzeugungseinrichtung (37) erzeugte Information auf einer Anzeigetafel angezeigt werden kann.

6. Messsystem für Metallschmelzen nach Anspruch 1, wobei der in dem arrangierten Eintauchteilbereich (23) des Testers vorgesehene Metalldetektionssensor (27) eine LC-Oszillationsschaltung enthält, welche eine vorbestimmte Spannung ausgibt, aber die Spannung durch Reagieren auf einen Wirbelstrom reduziert, der in geschmolzenem Metall hervorgerufen wird, wenn sich die Schaltung diesem annähert.

7. Messsystem für Metallschmelzen nach Anspruch 1, wobei die in dem arrangierten Eintauchteilbereich (23) des Testers vorgesehene Messeinrichtung (24) ausgewählt einen oder mehrere von einem Temperatursensor (24a) zum Messen der Temperatur von geschmolzenem Metall, einem Sauerstoffsensor (24b) zum Messen des gelösten Sauerstoffs und/oder der gelösten Elemente, enthalten in geschmolzenem Metall, einem CD-Sensor, der die Erstarrungstemperatur von geschmolzenem Metall misst, und einem Probenehmer (24c) enthält.

8. Messsystem für Metallschmelzen nach Anspruch 1, wobei in einem vorbestimmten Bereich (d) des Testers eine Schlackenabtasteinrichtung (25) vorgesehen ist, die oberhalb von dem oberen Ende (U) des arrangierten Eintauchteilbereichs (23) mit einem Abstand (dx) lokalisiert ist, der unter der Bedingung von dx<t in Bezug auf eine Dicke (t) der geschmolzenen Schlackenschicht (13) ausgebildet ist, wodurch die Schlackenabtasteinrichtung (25) in die geschmolzene Schlackenschicht (13) bei einer Position separat oberhalb von der Metallschmelzenhöhe (ML) mit dem Abstand (dx) eingetaucht ist, in einem Zustand, in welchem der Tester in geschmolzenem Metall eingetaucht ist, um zu veranlassen, dass das obere Ende (U) des arrangierten Eintauchteilbereichs (23) mit der Metallschmelzenhöhe (ML) übereinstimmt.

## Revendications

1. Système de mesure du métal en fusion dans lequel les éléments de métal en fusion à mesurer le sont dans une zone de profondeur prédéterminée (D) à partir d'un niveau de métal (ML) de métal en fusion (13) sur lequel se trouve une couche de scories (12), et dans lequel ledit système comprend une sonde (16) qui comprend une extrémité inférieure (16b) ayant une partie immergée (23) prévue pour être immergée dans ladite zone de profondeur prédéterminée (D), une extrémité supérieure (16a) prévue pour se prolonger au-dessus de ladite couche de scories (12) lorsque la sonde est immergée, des moyens de mesure (24) prévus dans ladite partie d'immersion aménagée (23) de mesure des éléments de métal en fusion à mesurer, et un capteur de détection de métal (27) positionné à un emplacement situé au-dessous de l'extrémité supérieure (U) de la zone de profondeur prédéterminée (D) à une distance (Dx),
une sous-lance (15) connectée à l'extrémité supérieure (16a) de ladite sonde (16) et avec laquelle ladite sonde (16) se déplace vers le bas à une vitesse (V) et remonte après avoir stoppé le mouvement vers le bas, un dispositif d'entraînement (17) permettant à ladite sous-lance (15) de monter et de descendre, et
un dispositif de commande (18) comportant un moyen(20) de commande de l'entraînement pour que ladite sous-lance (15) descende, s'arrête et remonte en utilisant ledit dispositif d'entraînement (18),
**caractérisé par le fait que** :
ledit système comprend également un dispositif de création de données (30) incluant un moyen pour déterminer la position d'arrêt (31) qui détermine, pendant le mouvement descendant de la sonde (16) un point de temps de détection (T1) et un point de temps d'arrêt (T2) qui arrive après l'écoulement d'une période de continuation de descente (Tx) (Tx=Dx/V) à partir dudit point de temps de détection (T1), un moyen d'émission d'un signal d'arrêt (32) qui envoie un signal d'arrêt (SS) audit moyen de commande d'entraînement (20) simultanément à l'arrivée d'un point de temps d'arrêt (T2), et
un moyen d'estimation (34) qui reçoit le signal de détection (DS) provenant dudit capteur de détection de métal (27)
et émet un signal de détermination de détection (JS) vers ledit moyen de détermination de la position d'arrêt (31), afin de déterminer ledit point de temps de détection (T1) ;
dans lequel ledit capteur de détection de métal (27) inclut un circuit qui alimente ledit moyen de d'estimation (34) avec une tension prédéterminée, dans lequel la tension est réduite par réaction avec le métal, de telle sorte que la tension soit réduite en réagissant avec la couche de scories liquides (12) et est encore réduite par réaction avec le métal en fusion (13) dont la réduction de la tension constitue ledit signal de détection (DS), et
le moyen d'estimation (34) reçoit ledit signal de détection (DS) provenant dudit capteur de détection de métal (27) et compare la réduction de sa tension avec un seuil prédéterminé, et ledit moyen d'estimation (34) envoie un signal de détermination de détection (JS) audit moyen de détermination de la position d'arrêt (31) uniquement lorsque la réduction de la tension dudit signal de détection (DS) est jugée comme dépassant ledit seuil selon lequel ledit moyen de détermination de la position d'arrêt (31) détermine ledit point de temps de détection (T1) ;
par conséquent, ledit moyen de détermination de la position d'arrêt (31) est constitué pour déterminer ledit point de temps de détection (T1) uniquement lorsque ledit capteur de détection de métal (27) atteint ledit niveau de métal (ML) du métal en fusion (13) après avoir traversé ladite couche de scories (12).

2. Système de mesure du métal en fusion selon la revendication 1, dans lequel un moyen de définition (35) est prévu dans ledit dispositif de création de données (30), de sorte que ledit moyen de définition (35) fixe des valeurs de ladite distance (Dx) et de ladite période de temps de descente (Tx), et les enregistre dans le moyen de détermination de la position d'arrêt (31).

3. Système de mesure du métal en fusion selon la revendication 1 ou 2, dans lequel ledit dispositif de création de données (30) prévoit un moyen de création de données de position du niveau (36) qui crée des informations concernant le niveau de métal du métal en fusion, en se basant sur ledit signal de détection (DS) du capteur de détection de métal (27).

4. Système de mesure du métal en fusion selon la revendication 1, 2 ou 3, dans lequel ledit dispositif de création de données (30) prévoit un moyen de création de données de position d'immersion (37) qui crée, en cherchant la distance entre le niveau de métal et la position d'immersion dudit moyen de mesure en se basant sur ledit point de temps d'arrêt (T2), des informations concernant la position de profondeur où lesdits éléments à mesurer ont été mesurés grâce au moyen de mesure.

5. Système de mesure du métal en fusion selon la revendication 3 ou 4, dans lequel un moyen d'affichage (38) est prévu dans ledit dispositif de création de données (30) dans lequel les informations créées par ledit moyen de création de données de position du niveau (36) ou par ledit moyen de création de données de position d'immersion (37) peuvent s'afficher sur un écran.

6. Système de mesure du métal en fusion selon la revendication1, dans lequel ledit capteur de détection du métal (27) prévu dans la partie d'immersion aménagée (23) de ladite sonde inclut un circuit oscillant LC fournissant la tension prédéterminée mais réduisant la tension par réaction à un courant de Foucault généré dans le métal en fusion lorsque le circuit s'en approche.

7. Système de mesure *du métal* en fusion selon la revendication 1, dans lequel ledit moyen de mesure (24) prévu dans la partie d'immersion aménagée (23) de ladite sonde inclut un ou plusieurs, choisi(s) à l'aide du capteur de température (24a) pour mesurer la température du métal en fusion, un détecteur d'oxygène (24b) pour mesurer l'oxygène dissous et/ou les éléments en solution contenus dans le métal en fusion, un capteur CD pour mesurer la température de solidification du métal en fusion, et un échantillonneur (24c).

8. Système de mesure *du métal* en fusion selon la revendication1, dans lequel un moyen d'échantillonnage des scories (25) est prévu dans une zone prédéterminée (d) de la sonde située au-dessus de l'extrémité supérieure (U) de ladite partie d'immersion aménagée (23) à une distance (dx), formé à la condition que dx<t par rapport à une épaisseur (t) de la couche de scories liquides (13), ce qui permet par conséquent d'immerger ledit moyen d'échantillonnage des scories (25) dans la couche de scories liquides (13), à un emplacement distinct au-dessus du niveau du métal en fusion (ML) à ladite distance (dx), dans un état dans lequel la sonde est immergée dans le métal en fusion de sorte que l'extrémité supérieure (U) de la partie d'immersion aménagée (23) coïncide avec le niveau de métal liquide (ML).
